# EUROPEAN PATENT SPECIFICATION

(11) **EP 2 892 517 B1**
(45) Date of publication and mention of the grant of the patent: **19.10.2016**
(21) Application number: 13762597.6
(22) Date of filing: 30.08.2013
(51) Int. Cl.: A61K 31/047, A61K 31/20, A61K 31/352, A61K 31/723, A61P 31/02, A61K 31/19, A61K 31/194

(54) **ANTIMICROBIAL COMPOSITION**
ANTIMIKROBIELLE ZUSAMMENSETZUNG
COMPOSITION ANTIMICROBIENNE

(30) Priority: 03.09.2012 NL 2009407
(43) Date of publication of application: 15.07.2015
(73) Proprietor: Dutch Renewable Energy B.V., 1398 CP Muiden (NL)
(72) Inventor: PELLIKAAN, Hubert Clemens, NL-3572 CA Utrecht (NL)
(74) Representative: Patentwerk B.V.
(86) International application number: PCT/NL2013/050628
(87) International publication number: WO 2014/035246

(56) References cited:
- EP-A2- 0 985 408
- EP-A2- 1 050 300
- WO-A1-2006/053170
- WO-A1-2011/061237
- WO-A1-2011/134802
- WO-A2-96/23479
- JP-A- 2012 097 037
- NL-C1- 1 024 714
- US-A- 5 234 703
- MURZYN ANNA ET AL: "Capric acid secreted by S. boulardii inhibits C. albicans filamentous growth, adhesion and biofilm formation.", PLOS ONE, vol. 5, no. 8, E12050, August 2010 (2010-08), pages 1-7, XP002688898, ISSN: 1932-6203
- ASHISH GUPTA ET AL: "Inhibition of adherence of multi-drug resistantby proanthocyanidin", UROLOGICAL RESEARCH ; A JOURNAL OF CLINICAL AND LABORATORY INVESTIGATION IN UROLITHIASIS AND RELATED AREAS, vol. 40, no. 2, 19 June 2011 (2011-06-19), pages 143-150, XP035029738, SPRINGER, BERLIN, DE ISSN: 1434-0879, DOI: 10.1007/S00240-011-0398-2

## Description

The present invention relates to an antimicrobial composition and a pharmaceutical formulation comprising the antimicrobial composition, a pharmaceutical formulation for treating or preventing the first stage in pathogenesis and the use of an organic acid as an anti-adhesion agent.

The throat is an open crossroad in the respiratory tract as well as a reservoir for many pathogens. A sore throat can be the starting point of many infections of the upper respiratory tract, e.g. otitis media and sinusitis, and the lower respiratory tract, e.g. bronchitis and pneumonia. In addition to the throat, also wounds can be a starting point of many infections. Adhesion and colonization of human tissue is known to be the first stage in pathogenesis of many infectious diseases.

A sore throat is initially mostly viral in nature, common viruses may provide an initial conditioning to the host mucosa that favours bacterial adherence which subsequently leads to bacterial biofilm formation. To overcome the adhesion of the pathogens to the host mucosa is a viable strategy in preventing infection. Additionally, the use of effective inhibitory agents may also exert a therapeutic advantage by inhibiting extended colonization and restricting the spread of infection. Causes of a sore throat vary from having a dry throat to severe throat infections like laryngitis. An effective composition must at least soothe the throat and take away the cause or causes of the sore throat.

The present invention therefore aims to provide an antimicrobial composition for treating or preventing the first stage in pathogenesis in order to prevent infections.

The present invention thereto provides an antimicrobial composition comprising at least one free fatty acid or its derivative and/or pharmaceutically acceptable salt thereof, at least one carboxylic acid or a pharmaceutically acceptable salt thereof and/or at least one carbohydrate or a pharmaceutically acceptable salt thereof as defined in the claims. It was found that a composition comprising a combination of at least one free fatty acid and at least one carboxylic acid having excellent disinfecting properties, i.e. exhibiting antiviral and antibacterial properties. It was further found that the combination of at least one free fatty acid and at least one carbohydrate, optionally in combination with at least one carboxylic acid, exerts a dual antimicrobial effect. The carbohydrate as well as the free fatty acid block the adhesion of the bacterial cells to the throat tissue and therefore exhibit good anti-adhesion properties. The free fatty acid and carboxylic acid exerts a potent microbicidal effect on contact with a microbial cell by dissolution of the lipid components of the microbial cell membrane.

The free fatty acids may be selected from saturated free fatty acid, unsaturated free fatty acid and combinations thereof, having from 6 to 10 carbon atoms are well soluble and exhibits good anti-adhesion properties and exerts potent microbicidal effect on contact with a microbial cell in case the free fatty acids are solubilised in a medium having a pH in the range of about 5.5 to about 7.0. Most preferred the free fatty acid having from 8 to 10 carbon atoms because free fatty acids with chain lengths varying from 8 to 10 carbon atoms show to have more strongly antiviral and antibacterial properties, i.e. inhibition of bacterial growth, compared to free fatty acids with chain lengths outside the most preferred range.

Preferred free fatty acids suitable for use in the antimicrobial composition of the present invention are selected from enanthic acid, caproic acid, caprylic acid, pelargonic acid, capric acid, and combinations thereof, and pharmaceutically acceptable salts and esters thereof. More preferred the free fatty acid of the present invention is selected from caprylic acid, pelargonic acid, capric and combinations thereof. Most preferred the free fatty acid of the present invention is selected from caprylic acid, capric acid and combinations thereof, and pharmaceutically acceptable salts and esters thereof, due to the more strongly antiviral and antibacterial properties compared to the other free fatty acids.

In addition to the antiviral and antibacterial properties of the free fatty acids of the present invention it was also found that the free fatty acids exhibit anti-adhesion properties. In particular free fatty acids selected from caprylic acid, capric acid and combinations thereof exhibit good anti-adhesion properties, i.e. inhibition adhesion of pathogenic bacteria to the human epithelium.

The carboxylic acid may be selected from alpha hydroxy acids, diacids, polyacids and combinations thereof. Preferably the carboxylic acid may be selected from glycolic acid, lactic acid, citric acid, mandelic acid, oxalic acid, malonic acid, succinic acid, glutaric acid, tartaric acid, adipic acid, pimelic acid, malic acid, maleic acid, fumaric acid and combinations thereof. It was found that the carboxylic acids present in the composition of the present invention exhibit excellent antiviral and antibacterial properties at a neutral pH, i.e. in the range of about 5.5 to about 7.0.

It was even further found that carboxylic acids exhibit excellent antiviral and antibacterial properties in low amounts within the range of about 0.01% to about 4% by weight of the total weight of the composition. Preferably, the concentration of the carboxylic acids is about 0.1% to about 3% by weight of the total weight of the composition, more preferred the concentration is about 0.2% to about 2% by weight of the total weight of the composition, even more preferred the concentration is about 0.3% to about 1% by weight of the total weight of the composition and most preferred the concentration is about 0.4% to about 0.5% by weight of the total weight of the composition. In a preferred embodiment, the carboxylic acid is selected from citric acid or a pharmaceutically acceptable salt thereof. It was found that a composition of the present invention comprising citric acid was effective against E. coli.

The carbohydrate used in the antimicrobial composition of the present invention is selected from a hydrogenated carbohydrate, a monosaccharide, a disaccharide, a polysaccharide and combinations thereof. In particular, the carbohydrate is preferably selected from monosaccharides and/or disaccharides including for example glucose, fructose, galactose, ribose, sucrose, maltose, lactose, cellulose, starch, glycogen, xylitol, mannitol, sorbitol, erythritol, mannose, D-mannose and combinations thereof. It was found that monosaccharides and/or disaccharides exhibit good anti-adhesion properties. More preferably the carbohydrates used in the present invention are selected from sucrose, xylitol, mannitol and combinations thereof exhibit excellent anti-adhesion properties.

In one embodiment of the present invention, the antimicrobial composition comprises a free fatty acid being caprylic acid and a carbohydrate being xylitol. It was found that the free fatty acid e.g. caprylic acid, exhibits clear anti-adhesion properties and that in combination with the carbohydrate, e.g. xylitol, an additional effect is provided on the adhesion inhibition.

In a further embodiment of the present invention, the antimicrobial composition comprises a carbohydrate selected from monosaccharides and/or disaccharides in combination with a carbohydrate selected from hydrogenated carbohydrates and/or polysaccharides, e.g. dextran, dextran sulphate, hyaluron, mannan, manuka honey and combinations thereof. It was found that a combination of two or more carbohydrates selected from a monosaccharide, e.g. xylitol, and polysaccharides, e.g. manuka honey and dextran, exhibits excellent anti-adhesion properties. In one embodiment of the present invention, the antimicrobial composition comprises a free fatty acid being caprylic acid and carbohydrates being xylitol, manuka honey, dextran or combinations thereof. It was found that the free fatty acid, e.g. caprylic acid, exhibits clear anti-adhesion properties and that in combination with the carbohydrates, e.g. xylitol and dextran, an additional effect is provided on the adhesion inhibition.

In another embodiment of the present invention, the antimicrobial composition further comprising at least one flavonoid or a pharmaceutically acceptable salt thereof. Preferably the flavonoid is selected from flavanols, flavones, anthocyanidins, isoflavonoids, neoflavonoids and combinations thereof. Most preferred the flavonoid is a proanthocyanidin. Also flavonoid-comprising components might be used. An example of a flavonoid-comprising component is Echinacea Purpurea extract.

The antimicrobial composition may further comprise at least one flavouring agent, viscosity-enhancing agent, emulsifying agent, acidity-regulating agent, humectant and/or preservative. The amount of additional agents used is preferably restricted, because it was found that the addition of several agents reduces the antimicrobial activity of the antimicrobial composition.

The antimicrobial composition may comprise at least one flavouring agent. Preferably the flavouring agent is selected from peppermint oil, candy flavour, sodium cyclamate and combinations thereof. The antimicrobial composition of the present invention can be used without comprising a flavouring agent.

The antimicrobial composition may comprise at least one viscosity-enhancing agent, i.e. thickening agent. Preferably the viscosity-enhancing agent is selected from xanthan gum, alginic acid, agar, carrageenan, locust bean gum, pectin, cellulose derivatives, gelatin and combinations thereof.

The antimicrobial composition may comprise at least one emulsifying agent, e.g. surfactant. Preferably the emulsifying agent is selected from polysorbate (Tween) 20, polysorbate 40, polysorbate 60, polysorbate 80, polyoxyethylene glycol alkyl ethers, glucoside alkyl ethers, polyoxyethylene glycol octylphenol ethers, polyoxyethylene glycol alkylphenol ethers, glycerol alkyl esters, poloxamers, polyoxyl castor oil, and combinations thereof. More preferably the emulsifying agent is polysorbate 80.

The antimicrobial composition may comprise at least one acidity-regulating agent, i.e. pH control agent. Preferably the acidity-regulating agent is selected from lactic acid, acetic acid, citric acid, adipate, tartaric acid, malic acid, fumaric acid and combinations thereof.

The antimicrobial composition may comprise at least one humectant, i.e. a desiccant. Preferably the humectant is selected from propylene glycol, hexylene glycol, butylene glycol, glyceryl triacetate, vinyl alcohol, neoagarobiose, sugar polyols such as glycerol, sorbitol, xylitol, xanthan gum and maltitol, polymeric polyols like polydextrose, quillaia, lactic acid, urea, glycerine, aloe vera gel, MP Diol, alpha hydroxy acids like lactic acid, honey and combinations thereof.

Preservatives may be used to prevent decomposition and to ensure microbiologic stability. Preservatives may be selected from benzethonium chloride, benzisothiazolinone, biphenyl, tert-butylhydroquinone, calcium tartrate, cetylpyridinium chloride, chloroacetamide, choji oil, dimethyl dicarbonate, ethylenediaminetetraacetic acid, germaben, glycolic acid, hexamethylenetetramine, imidazolidinyl urea, iodopropynyl butylcarbamate, isothiazolinone, lactic acid, methylchloroisothiazolinone, methylisothiazolinone, natamycin, nisin, parabens, persulfate, 2-phenylphenol, phytic acid, polyaminopropyl biguanide, benzoate salts, e.g. sodium benzoate, sorbate salts, e.g. potassium sorbate, chloride salts, e.g. sodium chloride, nitrate salts, e.g. sodium nitrate, quaternium-15, sorbic acid, sulfur dioxide, tiabendazole and combinations thereof.

The components of the antimicrobial composition are dissolved in an aqueous medium. The aqueous medium has a pH in the range of about 5.5 to about 7.0. Most preferred the aqueous medium has a pH in the range of about 6.0 to about 6.5. It was found that the free fatty acids of the present invention are sufficiently soluble in the aqueous medium having a pH in the range of about 6.0 to about 6.5 and wherein the free fatty acids, optionally in combination with the carboxylic acids, exhibits antiviral and antibacterial properties. An aqueous medium having a pH lower than about 5.5 is no longer suitable for solubilising the free fatty acids of the present invention. It was found that an aqueous medium having a pH higher than about 7.0 results in an antimicrobial composition with reduced antiviral and antibacterial properties.

The term "about" as used herein is intended to include values, particularly within 10% of the stated values.

In one embodiment of the present invention, the antimicrobial composition as described above is dissolved in an aqueous medium substantially free of multivalent metal ions, e.g. divalent metal ions such as calcium and magnesium ions, having a pH of about 6.0 to about 6.5. In another embodiment of the present invention, the antimicrobial composition as described above is at least partially dissolved in an aqueous medium comprising multivalent metal ions, e.g. calcium ions, having a pH of about 6.0 to about 6.5. In the presence of multivalent metal ions some of the free fatty acids used in the antimicrobial composition of the present invention precipitate in the form of a multivalent metal ion-free fatty acid complex. The complex formed still exhibits antiviral and antibacterial properties.

The antimicrobial composition of the present invention may further comprise sequestrants complexing the metal ions, e.g. calcium ions, from the solution. Preferably sequestrants may be selected from calcium disodium ethylene diamine tetra-acetate, glucono delta-lactone, sodium gluconate, potassium gluconate, sodium tripolyphosphate, sodium hexametaphosphate and combinations thereof.

It was further found that the amount of surfactants in the antimicrobial composition must be as low as possible to maximize the antiviral and antibacterial properties of the free fatty acid. Preferably the amount of surfactants is less than 10% by weight of the total weight of free fatty acid present in the antimicrobial composition. More preferably the amount of surfactants is less than 8% by weight, less than 6% by weight. Most preferred the amount of surfactant is about 4% by weight of the total weight of free fatty acid present in the antimicrobial composition.

In another aspect of the present invention, a pharmaceutical formulation comprising the antimicrobial composition of the present invention is provided. Preferably the pharmaceutical formulation is in the form of a drink, gel, drops or spray. Even more preferred the pharmaceutical formulation is in the form of a throat spray, mouthwash, nasal spray, eardrops, wound spray or wound gel. The antimicrobial composition may also be formulated in the form of a syrup.

In even another aspect of the present invention, a pharmaceutical formulation comprising the antimicrobial composition of the present invention is provided for treating or preventing the first stage in pathogenesis in order to prevent infections. Preferably the pharmaceutical formulation comprising the antimicrobial composition of the present invention is provided for treating or preventing a sore throat and, as a consequence, preventing respiratory tract infections. Also preferred the pharmaceutical formulation comprising the antimicrobial composition of the present invention is provided for treating or preventing otitis media or outer ear infection. The pharmaceutical formulation comprising the antimicrobial composition of the present invention is provided for treating or preventing wound infection diseases.

In particular a pharmaceutical formulation comprising the antimicrobial composition of the present invention is provided for preventing or reducing the adhesion of bacteria and other microorganism selected from gram-positive bacteria, e.g. Staphylococcus aureus, β-hemolytic Streptococcus including group A. Streptococcus pyogenes, γ-hemolytic Streptococcus including group C. Streptococcus faecalis and/or α-hemolytic Streptococcus including Streptococcus pneumoniae, gram-negative bacteria, e.g. Escherichia coli, Enterobacter aerogenes, Enterobacter and/or Haemophilus influenzae, Candida albicans and combinations thereof.

The pharmaceutical formulation comprising the antimicrobial composition of the present invention may also be used for the treatment of second and additional stages in the pathogenesis and/or treating multiple pathogenetical processes simultaneously.

The invention will now be further illustrated with reference to the following examples.

### Examples

4 different compositions were produced. The ingredients and amounts of the 4 different compositions are provided in table 1. Caprylic acid was dissolved in 50 kg water and the solution was adjusted to a pH of 6.5 by adding a mixture of lactic acid (90%) and sodium hydroxide (20%). Some precipitation of caprylic acid was allowed. The mixture formed was used as composition C.

**Table 1. Compositions including weight-%**

| Composition | A | B | C |
|---|---|---|---|
| Sodium Caprylate | 1.27 | 1.27 | 1.27 |
| Caprylic acid | - | - | - |
| Xylitol | 10.00 | 10.00 | - |
| Dextran 40 kD | 1.00 | 1.00 | - |
| Manuka Honey | 0.10 | 0.10 | - |
| Whey protein | - | - | - |
| Proanthocyanidin | 0.04 | - | - |
| Flavouring agent | 0.04 | 0.04 | - |
| Sodium cyclamate | 0.8 | 0.8 | - |
| Xanthan gum | 0.25 | 0.25 | - |
| Polysorbate 80 | 0.05 | 0.05 | - |
| Sodium benzoate | 0.04 | 0.04 | - |
| Potassium sorbate | 0.15 | 0.15 | - |
| Desiccant | - | - | - |
| Lactic acid / sodium hydroxide | qs ad pH 6.5 | qs ad pH 6.5 | qs ad pH 6.5 |
| Water | ad 100 | ad 100 | ad 100 |

For the preparation of compositions A and B, 750 L water was added to the caprylic acid solution and the mixture was stirred. A separate mixture of 10 kg comprising xylitol, dextran 40 kD, manuka honey, sodium cyclamate, xanthan gum, sodium benzoate and potassium sorbate was blended and added to the mixture and stirred for 10 minutes. 53 kg of xylitol was added and the bulky mixture was stirred for an additional 15 minutes. Additionally, a separate mixture of proanthocyanidin (only composition A), flavouring agent and polysorbate 80 was mixed. 3 L of the bulky mixture was added to the flavouring agent-polysorbate 80 mixture and firmly stirred. The firmly stirred suspension is added to the bulky mixture and the mixture was stirred for 15 minutes. The pH of the mixture was checked and if necessary adjusted with lactic acid (90%) or sodium hydroxide (20%) to a pH of 6.5.

3 ml of composition A was transferred into a well of a polystyrene 6-wells plate. The 6-wells plate was incubated for 30 min at 35-37°C while shaking (±150 rpm). Subsequently, composition A was removed from the well and 3 ml 10⁵-10⁶ cfu/ml microorganism (Candida Albicans ATCC 10231) suspension was added to the well. The microorganisms were allowed to adhere for 2h at 35-39°C while shaking (±150 rpm). The well was washed twice with water to removed non-adhere cells. The adhered cells were stained with 3 ml crystal violet solution. The well was washed three times with water to remove excess of the crystal violet solution. The adhered cells were examined using a microscope.

The above-described procedure was repeated for composition B and composition C.

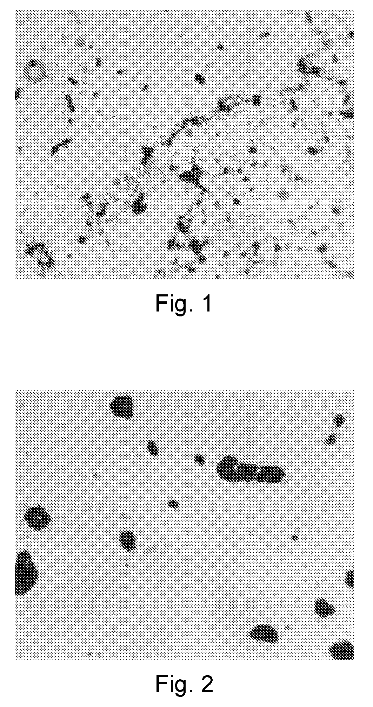

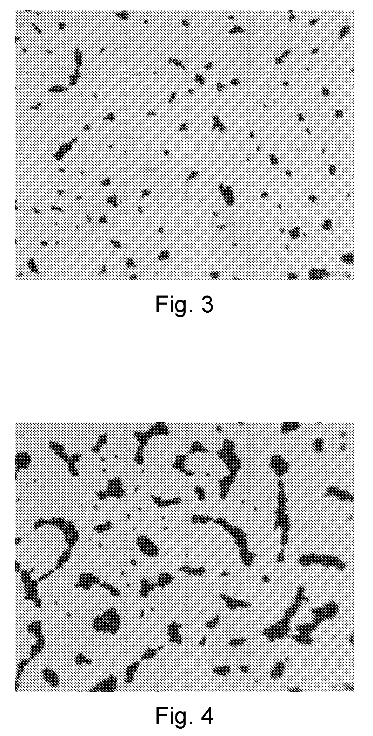

The percentage black on microscopic pictures taken from a bottom of a well treated with composition A (figure 1), composition B (figure 2) or composition C (figure 3) was compared to the percentage black on microscopic pictures taken from the bottom of a non-treated well (positive control; figure 4). Table 2 provides an overview of the comparative study.

**Table 2. Comparative study adhesion of C. Albicans ATCC 10231**

| Composition | Average % black on microscopic pictures of adhered C. albicans ATCC 10231* | Inhibition of adhesion compared to positive control (%) |
|---|---|---|
| Positive control | 10.86 | - |
| A | 4.42 | 59.3 |
| B | 4.02 | 63.0 |
| C | 5.98 | 44.9 |

| | | |
|---|---|---|
| *Average from three microscopic pictures | | |

Compositions A and B were also tested against Streptococcus pyogenes ATCC 19615 and Staphylococcus aureus ATCC 6538. Table 3 and 4 provide an overview of the results of the comparative study.

In order to test the antibacterial effect of Compositions A and B, sterile flasks were filled aseptically with 10 g of Compositions A or B. The flasks are inoculated with Streptococcus pyogenes ATCC 19615 or Staphylococcus aureus ATCC 6538 to give a final microbial suspension of 10⁵ to 10⁶ cfu per mL.

The flasks are stored at 20-25°C and at t=0 and 1 h, 1 g samples are taken from each flask to determine the number of viable microorganisms by plate count method. The results are provided in table 3 and 4.

**Table 3. Comparative study microbicidal effect compositions A and B against S. pyogenes**

| Composition | CFU count (¹⁰log) | | ¹⁰log reduction |
|---|---|---|---|
| | t=0 | t = 1 hour | |
| A | 5.9 | <1.0 | 4.9 |
| B | 5.9 | 2.1 | 3.9 |

**Table 4. Comparative study microbicidal effect compositions A and B against S. aureus**

| Composition | CFU count (¹⁰log) | | ¹⁰log reduction |
|---|---|---|---|
| | t=0 | t = 1 hour | |
| A | 5.4 | 2.8 | 2.6 |
| B | 5.4 | 3.2 | 2.2 |

Furthermore, the antibacterial effect of compositions comprising combinations of free fatty acids and carboxylic acids and derivatives were tested as well against Streptococcus pyogenes ATCC 19615 and Staphylococcus aureus ATCC 6538. The compositions D, E, F and G (see: table 5) were prepared by a similar method as given above. The pH of the compositions was adjusted to 6.0. The test results showed that compositions D, E, F and G exhibit antibacterial properties against S. pyogenes and S. aureus.

**Table 5. Compositions including weight-%**

| Composition | D | E | F | G |
|---|---|---|---|---|
| Caprylic acid | 1.00 | 1.00 | 4.00 | 2.00 |
| Caprylic acid monoglyceride | 1.00 | - | - | - |
| Sodium citrate | 1.00 | 1.00 | 4.00 | 4.00 |
| Eugenol | - | - | 0.10 | - |
| Flavouring agent | 0.50 | 0.50 | 0.50 | 0.50 |
| Sodium cyclamate | 0.80 | 0.80 | 0.80 | 0.80 |
| Xanthan gum | 0.25 | 0.25 | 0.25 | 0.25 |
| Sodium benzoate | 0.12 | 0.12 | 0.12 | 0.12 |
| Sodium hydroxide | 0.28 | 0.28 | 1.11 | 0.28 |
| Water | 95.06 | 96.06 | 89.12 | 92.06 |

In addition the antibacterial effect of compositions H, I and J (see: table 6) comprising combinations of free fatty acids and carboxylic acids were further tested against S. aureus, E. aerogenes, E. coli, C. albicans and A. brasiliensis. The results are shown in table 7 indicating the excellent antibacterial effect of the compositions.

**Table 6. Compositions including weight-%**

| Composition | H | I | J |
|---|---|---|---|
| Sodium Caprylate | 1.27 | 1.27 | 1.27 |
| Citric acid | 0.2 | 0.1 | 0.4 |
| Xylitol | 10.00 | 10.00 | 10.00 |
| Dextran 40 kD | 1.00 | 1.00 | 1.00 |
| Manuka Honey | 0.10 | 0.10 | 0.10 |
| Flavouring agent | 0.04 | 0.04 | 0.04 |
| Sodium cyclamate | 0.8 | 0.8 | 0.8 |
| Xanthan gum | 0.25 | 0.25 | 0.25 |
| Polysorbate 80 | 0.05 | 0.05 | 0.05 |
| Sodium benzoate | 0.04 | 0.04 | 0.04 |
| Potassium sorbate | 0.15 | 0.15 | 0.15 |
| Lactic acid / sodium hydroxide | qs ad pH 6.5 | qs ad pH 6.5 | qs ad pH 6.5 |
| Water | ad 100 | ad 100 | ad 100 |

**Table 7. Microbicidal effect of compositions H, I and J against various microorganisms**

| Microorganism | CFU count | | | | | | |
|---|---|---|---|---|---|---|---|
| | t=0 | H | | I | | J | |
| | | t = 1h | t = 24h | t = 1h | t = 24h | t = 1h | t = 24h |
| S. aureus | 10⁶ | <10 | <10 | <10 | <10 | <10 | <10 |
| E. aerogenes | 10⁶ | <10 | <10 | <10 | <10 | <10 | <10 |
| E. Coli | 10⁶ | <10 | <10 | <10 | <10 | <10 | <10 |
| C. albicans | 10⁶ | <10 | <10 | <10 | <10 | <10 | <10 |
| A. brasiliensis | 10⁶ | <10 | <10 | <10 | <10 | <10 | <10 |

## Claims

1. Antimicrobial composition comprising:
a) at least one free fatty acid or a pharmaceutically acceptable salt thereof, wherein the free fatty acid is selected from a free fatty acid having from 6 to 10 carbon atoms;
b) at least one carboxylic acid or a pharmaceutically acceptable salt thereof selected from an alpha hydroxyl acid; and
c) optionally, at least one carbohydrate or a pharmaceutically acceptable salt thereof selected from a hydrogenated carbohydrate, a monosaccharide, a disaccharide, a polysaccharide and combinations thereof,
wherein the composition is dissolved in an aqueous medium having a pH in the range of about 5.5 to about 7.0.

2. Antimicrobial composition comprising:
a) at least one free fatty acid or a pharmaceutically acceptable salt thereof, wherein the free fatty acid is selected from a free fatty acid having from 6 to 10 carbon atoms;
b) at least one carboxylic acid or a pharmaceutically acceptable salt thereof selected from an alpha hydroxyl acid, a diacid, a polyacid and combinations thereof; and
c) at least one carbohydrate or a pharmaceutically acceptable salt thereof selected from a hydrogenated carbohydrate, a monosaccharide, a disaccharide, a polysaccharide and combinations thereof,
wherein the composition is dissolved in an aqueous medium having a pH in the range of about 5.5 to about 7.0.

3. Antimicrobial composition according to claim 1 or 2, wherein the free fatty acid is selected from caprylic acid, capric acid and combinations thereof, and pharmaceutically acceptable salts and esters thereof.

4. Antimicrobial composition according to any of the preceding claims, wherein the carboxylic acid is citric acid.

5. Antimicrobial composition according to any of the preceding claims, wherein the amount of carboxylic acid is in the range of about 0.01% to about 4% by weight of the total weight of the composition.

6. Antimicrobial composition according to any of the preceding claims, wherein the carbohydrate is selected from sucrose, xylitol, mannitol and combinations thereof.

7. Antimicrobial composition according to any of the preceding claims, wherein the organic acid is caprylic acid and the carbohydrate is xylitol.

8. Antimicrobial composition according to any of the preceding claims, wherein the composition further comprises at least one flavonoid or a pharmaceutically acceptable salt thereof.

9. Antimicrobial composition according to claim 8, wherein the flavonoid is a proanthocyanidin.

10. Antimicrobial composition according to any of the preceding claims, wherein the composition further comprises a surfactant, and wherein the amount of surfactant is less than 10% by weight of the total weight of free fatty acid present, preferably about 4% by weight of the total weight of free fatty acid present.

11. Antimicrobial composition according to any of the preceding claims, wherein the aqueous medium has a pH in the range of about 6.0 to about 6.5.

12. Pharmaceutical formulation comprising the composition of any of the preceding claims, wherein the formulation is in the form of a drink, gel, drops or spray.

13. Pharmaceutical formulation according to claim 12, wherein the formulation is in the form of a throat spray, mouthwash, nasal spray, ear drops, wound spray or wound gel.

14. Pharmaceutical formulation according to claims 12 or 13 for treating or preventing the first stage in pathogenesis.

15. Pharmaceutical formulation according to claims 12 or 13 for preventing or reducing the adhesion of gram-positive bacteria, gram-negative bacteria and/or other microorganism to human tissue.

## Patentansprüche

1. Antimikrobielle Zusammensetzung, umfassend:
a) mindestens eine freie Fettsäure oder ein pharmazeutisch annehmbares Salz davon, wobei die freie Fettsäure aus einer freien Fettsäure mit 6 bis 10 Kohlenstoffatomen ausgewählt ist;
b) mindestens eine Carbonsäure oder ein pharmazeutisch annehmbares Salz davon, ausgewählt aus einer alpha-Hydroxysäure; und
c) gegebenenfalls mindestens ein Kohlenhydrat oder ein pharmazeutisch annehmbares Salz davon, ausgewählt aus einem hydrierten Kohlenhydrat, einem Monosaccharid, einem Disaccharid, einem Polysaccharid und Kombinationen davon,
wobei die Zusammensetzung in einem wässrigen Medium mit einem pH-Wert im Bereich von ungefähr 5,5 bis ungefähr 7,0 gelöst ist.

2. Antimikrobielle Zusammensetzung, umfassend:
a) mindestens eine freie Fettsäure oder ein pharmazeutisch annehmbares Salz davon, wobei die freie Fettsäure aus einer freien Fettsäure mit 6 bis 10 Kohlenstoffatomen ausgewählt ist;
b) mindestens eine Carbonsäure oder ein pharmazeutisch annehmbares Salz davon, ausgewählt aus einer alpha-Hydroxysäure, einer Disäure, einer Polysäure und Kombinationen davon; und
c) mindestens ein Kohlenhydrat oder ein pharmazeutisch annehmbares Salz davon, ausgewählt aus einem hydrierten Kohlenhydrat, einem Monosaccharid, einem Disaccharid, einem Polysaccharid und Kombinationen davon,
wobei die Zusammensetzung in einem wässrigen Medium mit einem pH-Wert im Bereich von ungefähr 5,5 bis ungefähr 7,0 gelöst ist.

3. Antimikrobielle Zusammensetzung nach Anspruch 1 oder 2, wobei die freie Fettsäure aus Caprylsäure, Caprinsäure und Kombinationen davon sowie pharmazeutisch annehmbaren Salzen und Estern davon ausgewählt ist.

4. Antimikrobielle Zusammensetzung nach einem der vorhergehenden Ansprüche, wobei es sich bei der Carbonsäure um Zitronensäure handelt.

5. Antimikrobielle Zusammensetzung nach einem der vorhergehenden Ansprüche, wobei die Menge an Carbonsäure im Bereich von ungefähr 0,01 Gew.-% bis ungefähr 4 Gew.-% des Gesamtgewichts der Zusammensetzung liegt.

6. Antimikrobielle Zusammensetzung nach einem der vorhergehenden Ansprüche, wobei das Kohlenhydrat aus Saccharose, Xylit, Mannit und Kombinationen davon ausgewählt ist.

7. Antimikrobielle Zusammensetzung nach einem der vorhergehenden Ansprüche, wobei es sich bei der organischen Säure um Caprylsäure und bei dem Kohlenhydrat um Xylit handelt.

8. Antimikrobielle Zusammensetzung nach einem der vorhergehenden Ansprüche, wobei die Zusammensetzung weiterhin mindestens ein Flavonoid oder ein pharmazeutisch annehmbares Salz davon umfasst.

9. Antimikrobielle Zusammensetzung nach Anspruch 8, wobei es sich bei dem Flavonoid um ein Proanthocyanidin handelt.

10. Antimikrobielle Zusammensetzung nach einem der vorhergehenden Ansprüche, wobei die Zusammensetzung weiterhin ein Tensid umfasst, und wobei die Menge an Tensid weniger als 10 Gew.-% des Gesamtgewichts der vorhandenen freien Fettsäure, vorzugsweise ungefähr 4 Gew.-% des Gesamtgewichts der vorhandenen freien Fettsäure, ausmacht.

11. Antimikrobielle Zusammensetzung nach einem der vorhergehenden Ansprüche, wobei das wässrige Medium einen pH-Wert im Bereich von ungefähr 6,0 bis ungefähr 6,5 aufweist.

12. Pharmazeutische Formulierung, die die Zusammensetzung nach einem der vorhergehenden Ansprüche umfasst, wobei die Formulierung in Form eines Getränks, eines Gels, von Tropfen oder eines Sprays vorliegt.

13. Pharmazeutische Formulierung nach Anspruch 12, wobei die Formulierung in Form eines Hals-Sprays, eines Mundwassers, eines Nasen-Sprays, von Ohrentropfen, eines Wund-Sprays oder eines Wund-Gels vorliegt.

14. Pharmazeutische Formulierung nach den Ansprüchen 12 oder 13 zum Behandeln oder Vorbeugen des ersten Stadiums von Pathogenese.

15. Pharmazeutische Formulierung nach den Ansprüchen 12 oder 13 zum Vorbeugen oder Vermindern des Haftens von grampositiven Bakterien, gramnegativen Bakterien und/oder eines anderen Mikroorganismus an menschlichem Gewebe.

## Revendications

1. Composition antimicrobienne comprenant :
a) au moins un acide gras libre ou un sel de qualité pharmaceutique de celui-ci, dans laquelle l'acide gras libre est choisi parmi les acides gras libres comportant de 6 à 10 atomes de carbone ;
b) au moins un acide carboxylique ou un sel de qualité pharmaceutique de celui-ci choisi parmi les acides alpha-hydroxylés ; et
c) éventuellement, au moins un glucide ou un sel de qualité pharmaceutique de celui-ci, choisi parmi un glucide hydrogéné, un monosaccharide, un disaccharide, un polysaccharide et les combinaisons de ceux-ci,
dans laquelle la composition est dissoute dans un milieu aqueux présentant un pH se situant dans une fourchette d'environ 5,5 à environ 7,0.

2. Composition antimicrobienne comprenant :
a) au moins un acide gras libre ou un sel de qualité pharmaceutique de celui-ci, dans laquelle l'acide gras libre est choisi parmi les acides gras libres comportant de 6 à 10 atomes de carbone ;
b) au moins un acide carboxylique ou un sel de qualité pharmaceutique de celui-ci choisi parmi un acide alpha-hydroxylé, un diacide, un polyacide et des combinaisons de ceux-ci ; et
c) au moins un glucide ou un sel de qualité pharmaceutique de celui-ci, choisi parmi un glucide hydrogéné, un monosaccharide, un disaccharide, un polysaccharide et les combinaisons de ceux-ci,
dans laquelle la composition est dissoute dans un milieu aqueux présentant un pH se situant dans une fourchette d'environ 5,5 à environ 7,0.

3. Composition antimicrobienne selon la revendication 1 ou 2, dans laquelle l'acide gras libre est choisi parmi l'acide caprylique, l'acide caprique et les combinaisons de ceux-ci, ainsi que les sels et esters de qualité pharmaceutique de ceux-ci.

4. Composition antimicrobienne selon l'une quelconque des revendications précédentes, dans laquelle l'acide carboxylique est l'acide citrique.

5. Composition antimicrobienne selon l'une quelconque des revendications précédentes, dans laquelle la quantité d'acide carboxylique se situe dans une fourchette d'environ 0,01 à environ 4 % en poids par rapport au poids total de la composition.

6. Composition antimicrobienne selon l'une quelconque des revendications précédentes, dans laquelle le glucide est choisi parmi le saccharose, le xylitol, le mannitol et les combinaisons de ceux-ci.

7. Composition antimicrobienne selon l'une quelconque des revendications précédentes, dans laquelle l'acide organique est l'acide caprylique et le glucide est le xylitol.

8. Composition antimicrobienne selon l'une quelconque des revendications précédentes, dans laquelle la composition comprend, en outre, au moins un flavonoïde ou un sel de qualité pharmaceutique de celui-ci.

9. Composition antimicrobienne selon la revendication 8, dans laquelle le flavonoïde est une proanthocyanidine.

10. Composition antimicrobienne selon l'une quelconque des revendications précédentes, dans laquelle la composition comprend, en outre, un tensioactif, et dans laquelle la quantité de tensioactif est inférieure à 10 % en poids par rapport au poids total d'acide gras libre présent et, de préférence, inférieure à environ 4 % en poids par rapport au poids total d'acide gras libre présent.

11. Composition antimicrobienne selon l'une quelconque des revendications précédentes, dans laquelle le milieu aqueux présente un pH se situant dans une fourchette d'environ 6,0 à environ 6,5.

12. Formulation pharmaceutique comprenant la composition selon l'une quelconque des revendications précédentes, dans laquelle la formulation se présente sous la forme d'une boisson, d'un gel, de gouttes ou d'un spray.

13. Formulation pharmaceutique selon la revendication 12, dans laquelle la formulation se présente sous la forme d'un spray pour la gorge, de bains de bouche, d'un spray nasal, de gouttes pour les oreilles, d'un spray pour les blessures ou d'un gel pour les blessures.

14. Formulation pharmaceutique selon la revendication 12 ou 13 pour le traitement ou la prévention du premier stade de la pathogenèse.

15. Formulation pharmaceutique selon la revendication 12 ou 13 pour la prévention ou l'inhibition de l'adhérence des bactéries à gram positif, des bactéries à gram négatif et/ou d'autres microorganismes aux tissus humains.
